(19) Europäisches Patentamt
European Patent Office
Office européen des brevets

(11) EP 3 398 607 A1

(12) **EUROPEAN PATENT APPLICATION**
published in accordance with Art. 153(4) EPC

(43) Date of publication:
**07.11.2018 Bulletin 2018/45**

(21) Application number: **16880236.1**

(22) Date of filing: **29.12.2016**

(51) Int Cl.:
***A61K 36/185*** *(2006.01)*

(86) International application number:
**PCT/CL2016/050081**

(87) International publication number:
**WO 2017/113032 (06.07.2017 Gazette 2017/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.12.2015 CL 20153798**

(71) Applicant: **Universidad de Santiago de Chile
Santiago (ES)**

(72) Inventors:
- **ZUÑIGA, Gustavo
Santiago (CL)**
- **FARAN, Ruth Paz
Santiago (CL)**
- **TAPIA ULLOA, Andrea
Santiago (CL)**
- **PARADA, Rodolfo
Santiago (CL)**

(74) Representative: **Pons Ariño, Angel
Pons Patentes y Marcas Internacional, S.L.
Glorieta Rubén Dario 4
28010 Madrid (ES)**

(54) **NATURAL ANTIBACTERIAL AND ANTIOXIDANT COMPOSITION OF PHENOL EXTRACTS FROM ARISTOTELIA CHILENSIS AND PRODUCTION METHOD**

(57) The present invention relates to a natural antibacterial and antioxidant composition of an extract rich in phenol compounds obtained from seedlings of *A. Chilensis,* also known as maqui, cultured *in vitro.* Specifically, a method is described for generating plant biomass in a sustainable manner, without needing to use materials grown in natural conditions, thereby reducing the impact on the exploitation of the species. The biomass generated is used to obtain a hydroalcoholic extract having antioxidant and antibacterial properties, owing to which the composition can be added directly to food products, cosmetics, pharmaceuticals for human consumption, or a combination of same.

EP 3 398 607 A1

## Description

### Field of the Invention

The present application relates to an antioxidant and a natural composition antibacterial phenolic compounds enriched extract obtained from seedlings of A. *chilensis,* maqui, cultured *in vitro.* Specifically, a method is described for generating plant biomass sustainably, without using materials grown under natural conditions, reducing the impact on the operation of the species. This biomass generated is used to obtain a hydroalcoholic extract, which has antioxidant and anti-bacterial properties, so it is possible to add the present composition directly to foodstuffs, cosmetics, pharmaceuticals for human consumption, or a combination thereof.

### Prior art

At present there is a great demand for natural products, for use in different fields of industry. For food, incorporating products that contribute to the maintenance and/or improvement of the health of those who consume them, originated the so-called functional foods, is a constant demand for products.

The lipidic oxidation of food, causes the decomposition of flavor generated the formation of potentially toxic products, discoloration of pigments and loss of nutritional value due to destruction of soluble vitamins and essential Graos acids (Nakatani, N (1997). Antioxidants, from spices and herbs In Natural Antioxidants: Chemistry, Health Effects and Applications Page 64-75 AOCS Press). Decomposition reactions of cellular macromolecules, are catalyzed by factors such as singlet oxygen, UV radiation, heat, metal.

A natural antioxidant is a substance capable of neutralizing the oxidizing action of free radicals by releasing electrons which without captured by free radicals fulfilling a preventive role in the development of aging and certain neurodegenerative diseases.

The antioxidant to interact with the free radical, gives it an electron weakening their action (Lourdes B., Garcia L, Red D., Zanches E., (2001) Plants with antioxidant properties Biomedica Cuban Journal Research 20: 231 - 235). Free radicals are atoms or groups of atoms having one unpaired electron or free, so they are very reactive, since they tend to capture an electron from stable molecules in order to achieve stability. Once the free radical captures an electron, the stable molecule that it yields becomes a new free radical, thus starting a chain reaction that can destroy the cell (M. Avello, Suwalsky M. (2006). Free radicals, natural antioxidants and protection mechanisms Athena 492: 161 -172).

Antioxidants to be present in foods or in the body at low concentrations compared to oxidizable substrate retard or prevent oxidation. Incorporation into food before processing to heat helps preserve product quality by delaying autoxidation and development of racidity as well as discoloration and loss of nutrients. The inhibitory effects of the antioxidant are related to their ability to prevent chain initiation of antioxidants, by donating a hydrogen atom or an electron to the radical species (Dziezak, JD (1986) Preservatives. Antioxidants The ultimate answer Food Technol 40 to oxidation.. 94-97). The most widely used synthetic antioxidants are butilhidroxilanisol (BHA), butylated hydroxytoluene (BHT), propyl gallate (PG) and tert-butylhydroquinone (TBHQ).

Worldwide there is great interest in replacing synthetic antioxidants in food, due to unwanted side effects, by naturally occurring antioxidants (Chen et al 1992).

Phenolic compounds are widespread in futas and vegetables (Lopez, J., (2008) Functional foods. Significance and Applications Agricultural School Zamorano Pan Chile). Phenolic compounds are related to the sensory quality of plant foods. In addition these compounds act as natural antioxidants in foods, so obtaining and preparation of products with a high content of them involves a reduced use of synthetic antioxidants. From a nutritional standpoint antioxidant activity of phenolic compounds is associated with its protective role of cardiovascular disease and cancer. (Posada el at., 2003).

The main phenolic compounds present in plants are: phenolic acids, flavonoids and polyphenols. Plants contain a variety of phenolic compounds, including simple phenols, phenylpropanoids, benzoic acid derivatives, flavonoids, stybelnes, tannins, ligannos: In general these molecules actuvidad prsetan antibiotic, as natural pesticides, protectres UV (Shahidi, F. And Naczk, M (2004) Phenolics in the food and nutraceuticals London CRC Press Page 1 - 16).

Moreover also the food industry is constantly searching for compounds with antimicrobial activity, to be used as additives. Several studies have shown the capacity antibacterial phenolic compounds in products such as fruits and vegetables minimally processed (Gutierrez, J., Rodriguez, G., Barry-Ryan, C, & Bourke, P. (2008). Efficacy of plant), minced meat (Hulankova, R., Borilova, G., & Steinhauserova, I. (2013). Combined antimicrobial effect of oregano essential oil and caprylic acid in minced beef. meat Science, 95 (2), 190-194), chicken (Fernandez-Pan, I., Mendoza, M., & Mate, JI (2013). Whey protein Isolate edible films With essential oils incorporated to improve increase the microbial quality of poultry. J Sci Food Agric, 93 (12), 2986- 2994).

chilensis (Mol.) Stuntz is known vulgarly with the name of Machi. It is a native tree from the subantarctic forests of Chile and Argentina. Demarcations grows mainly in forest and beds of watercourses, always associated with other

species of greater importance. It is preferably carried out in wet soils of the central valley in the foothills of two mountain ranges, gorges or margins of forests, from sea level to 2,500 meters The maqui is distributed from the Region of Coquimbo to Aysen, it is even possible to find in the Juan Fernandez Archipelago (Rodriguez, R. 1995. Flora of Chile. Ed. Universidad de Concepción, Concepción. 408 p.). It has a great morphological plasticity, appearing as a shrub in the northernmost area of distribution and as a tree in the south.

In fruit has detected the presence of flavonoids with antioxidant capacity (Valdebenito, G. 2006. maqui Package Technology. Http://www.gestionforestal.cl. Forestry Institute (10.06.2012)) and low concentrations of alkaloids indole such as aristotelina, aristotelona, Aristona and aristotelinina. This is how the maqui has the highest level of antioxidants (ORAC) of all known fruits and measures by the US Brunswick Laboratories (Chile Foundation, 2012. http://www.fundacionchile.com/noticias/demanda-de-maqui-grows exponentially-enforma-. (04.05.2012)). The presence of these antioxidants in the fruit is through anthocyanidins, which would be responsible for the characteristic purple fruit (Silva, M. Bittner, M. 1992. Study of chemical species Eleocarpaceae family grown in Chile. in: Chemistry flora Orlando Muñoz Chile (ed.) University of Chile Santiago, Chile, pp. 153-166). Therefore, it is credited dyer use in handicrafts and even to enhance the color of red wine, a fact that is forbidden by the law of the Chilean wine industry.

In the previous art, no patent in which the properties described in this application are protected are reported nor described the use of in vitro culture of plant maqui tissue to obtain a composition of phenolics antioxidants and antibacterial properties.

## Brief Description of the Figures.

Fig. 1 shows a flask with seedlings of Maqui cultivated *in vitro.* These plantlets are the raw material for obtaining extracts. Maqui seedlings grown *in vitro.* The bottle shows a plantlet 30 days of culture in MS medium.

Fig. 2 shows the in vitro capacity antioxidant plants compared to fruits of maqui.

Fig. 3 shows the effect of the addition of extract maqui in antioxidante capacity canola oil. Antioxidant oil capacity canola extract supplemented maqui.

Fig. 4 shows the cytotoxicity maqui extract. Cytotoxic activity maqui extract.

Fig. 5 shows the antibacterial maqui extract activity on *Listeria monocytogenes, E. coli* and *S. Aureus.* Antibacterial activity maqui extract.

Fig. 6 metabolites present in the extract maqul. Analysis of *in vitro* 85% ethanol extract.

## Detailed Description of the invention.

The present application relates to a natural composition antibacterial and antioxidant and an extract enriched in phenolic compounds obtained from seedlings of *A. chilensis,* maqui, cultured *in vitro.* Specifically, a method is described for generating plant biomasa sustainably, without using grown under natural conditions material which reduces the impact on the operation of the species. This biomasa generated is used to obtain an extract hydroalcoholic, having antoxidant and antibacterial properties, which could be added directly to food or pharmaceutical products for human consumption, or both. Optionally, the extract can be encapsulated in cyclodextrins, maltodextrosas and acacia, using techniques known in the art.

In another optional form, the present composition is added directly into food, cosmetics, pharmaceutical products for human consumption in a proportion of between 0.01 and 0.5% by weight.

Example 1. Preparation of the ethanol extract of Maqui.

The present application relates to a natural antibacterial and antioxidant composition and an extract enriched phenolic compounds obtained from seedlings of *A. chilensis,* maqui, cultured *in vitro*. Specifically, a method is described for generating plant biomass sustainably, without using materials grown under natural conditions, reducing the impact on the operation of the species. This biomass generated is used to obtain a hydroalcoholic extract, which has antioxidant and antibacterial properties, so it could be added directly to food or pharmaceutical products for human consumption, or both.

1.1. Maqui seedling production *in vitro.*

*In vitro,* cultures were initiated from shoots of adult trees collected in the 7th region, commune of Licanten, Chile. Outbreaks collected from adult trees were taken in a wet paper bag to avoid dehydration of tissues. In the laboratory, the buds were sectioned into meristematic explants with buds. These explants were placed in a container with distilled water and 10 drops of the detergent Tween 20, for 10 minutes. The material was then transferred to a container with commercial sodium hypochlorite (20%), for 5 minutes. Then the explants were washed 3 times with sterile distilled water.
Clean explants were placed in vials with 20 mL of MS culture supplemented with vitamins (Murashige and Skoog). Then the explants were placed in a growth chamber at a temperature of 22 $\pm$ 2 ° C and 16 hour photoperiod light/8 hours dark. Bottles kept under these conditions for up to 45 days.

1.2. Obtaining maqui extract.

Maqui seedlings 20 to 45 days of culture *in vitro,* were used to obtain a hydroalcoholic extract. The concentration of ethanol used varies between 25 and 85% v/v. Extraction methodology was by diffusion or maceration in a temperature range between 20 and 70°C. The extract obtained was stored in amber bottles at room temperature.

Example 2. Determination of the antioxidant capacity of the extract maqui.

Ethanolic extracts maqui sheet, fruit and plant *in vitro,* at a concentration of 0.1 g fresh/ml ethanol tissue were used for the determination of antioxidant capacity. The antioxidant capacity was determined using the method of DPPH (Radical 2,2- diphenyl-1 -picrilhidrazil).
To determine the antioxidant activity of the extracts in vitro plantlet, fruit and leaf grown tree, they took 20 $\mu$L of each extract and was added to each dissolution $\mu$L 180 150 $\mu$mol/L of DPPH. All reactions were carried out for 10 minutes at room temperature, in 96 wells protected from light, after which, absorbance at 520 nm on a TECAN spectrophotometer Microplate Reader (Life Sciences) is measured. Within each block each treatment was applied 3 times.
Antioxidant activity was expressed as percentage inhibition which corresponds to the amount of DPPH radical neutralized extract at a concentration determined according to the following equation.

$$\% \text{ Inhibition (AA)} = (A- A1)/A$$

AA Inhibition I (1)
A = Absorbance white
A1 = Absorbance of Sample

The results showed that the *in vitro* seedlings maqui showed higher activity than fruits and similar to that of adults d tree leaves (ANOVA analysis a channel with n 6 p <0,05.) Fig. 2.

Example 3. Effect of the addition of extract maqui in canola oil.

Canola oil was used, extra virgin not supplemented with antioxidants. The ethanolic extract maqui seedlings in vitro, was dried in a rotary evaporator. The dry matter, were taken between 0.5 and 1 mg was, which were added to 1 mL of oil. At supplemented oils they were determined the antioxidant capacity by DPPH test described above.
Analysis of the antioxidant capacity of the oils was possible to determine that the dry extract maqui does increase the antioxidant capacity of canola oil. However, this increase was less than the value detected in commercial olive oils (Fig. 3).
Chart antioxidant capacity by DPPH canola oils supplemented with various concentrations of dry methanolic extract, antioxidant capacity compared to canola oil unsupplemented and olive oils. ANOVA one way, p <0.05.

Example 4. Cytotoxic activity of extracts maqui

Cytotoxicity of the ethanolic extracts of plants maqui in vitro was determined in a human cell line HEK 293 Cells were grown in DMEM (Dulbecco's Modified Eagle's Minimal) supplemented with streptomycin and 1% penicillin and Fetal Serum 10% bovine. To study cytotoxicity ethanolic extracts of plant maqui *in vitro* were taken to dryness and subjected to a solubility curve in DMEM and 0.2% DMSO. The solubility of the dry extracts in medium varies between 10 and 100 $\mu$g/mL. Cells were seeded in 96 - well plates at a concentration of $10^4$ cells/well. 100, 85, 65, 50, 30 and 10 $\mu$g/mL. After 24 h extracts at different concentrations were added. After this and once after 24 h, we proceeded to add

the dye Neutral Red. After 2 hrs the plates were measured in an ELISA-type detector at an absorbance of 540 nm. The experiments were performed in 3 separate plates and the extracts were added in triplicates.

The results obtained in HEK293 cell line showed that the percentage of cell death did not exceed 30% for any of the concentrations used, not showing significant differences a significance level of 95% and analyzed by ANOVA with a channel (Figure 4).

These results suggest that the plant extract maqui *in vitro* is not toxic to the cell line HEK293 therefore not present a potential toxicity for human consumption.

Additionally maquies extract was able to protect HEK 293 cells, induced oxidation of hydrogen peroxide. Cells were seeded in 96 - well plates at a concentration of $10^4$ cells/well. After 24 hrs, concentration of 10, 50 and 100 ug/mL of extract were added maqui. After 24 hrs, hydrogen peroxide was added at a concentration of 1 mM for 4 hrs, both control wells and wells containing the various concentrations of the extracts.

Results showed that the extract maqui protects HEK293 cells induced oxidation of hydrogen peroxide, in the entire range of concentrations used (Fig.5 and Table 1).

Table 1: Inhibition Halo maqui extract in bacteria.

| Extract | *E. coli* | *S. aureus* | *L. monocytogenes* |
|---|---|---|---|
| Maqui | 13,8 ± 2,1 | 12,1 ± 0,8 | 12,7 ± 0,6 |

Table 2: gentamycin resistance used bacteria

| Concentration, µg/ml | *E. coli* | | *S. aureus* | | *L. monocytogenes* | |
|---|---|---|---|---|---|---|
| | X | SD | X | SD | X | SD |
| 0,5 | 27,5 | 0,7 | 1,5 | 1,5 | 18,3 | 0,6 |
| 0,25 | 26 | 0 | 2,1 | 2,1 | 16,3 | 0,6 |
| 0,125 | 24 | 0 | 1,2 | 1,2 | 14,3 | 2,9 |
| 0,0625 | 23 | 0 | 0 | 0 | 14 | 1 |
| 0,03125 | 22 | 0 | 0 | 0 | 0 | 0 |

Example 5. Antibacterial activity of extracts maqui.

The antibacterial activity was determined using the test dilution Halo Petri plates 100 x 15 cms sterile, seeded with LB medium and allowed to cool to room temperature (Fig. 6). Then sterile Pasteur pipette agar drilled for wells where maqui extract was deposited. Dilutions of extract maqui were deposited in each well in a volume of 50 µl. Then he allowed to evaporate the solvent. This procedure was repeated until 350 uL complete extract. After this was plated on plates the respective bacteria tested: *Escherichia coli; Staphylococcus aureus; Listeria monocytogenes.*

Results showed that the extract maqui shows inhibitory ability of the bacteria tested (Fig.5)

Example 6. Analysis of compounds in the extract maqui by high resolution liquid chromatography coupled to a mass spectrometer (LC-MS).

The analysis of the compounds present in the extracts maqui is performed on a Agilent Technologies 1200 series HPLC coupled to a mass spectrometer Agilent Technologies 6410 Triple Quad (LCMS).

20 uL for analysis of the extracts were added to the separation column RP- May 18 um x 4.6 mm x 150 mm, Agilent, with a flow of 0.3 mL/min. The temperature was kept constant at 25 C. The conditions of mass detector was: electrospray ionisation pro (ESI) in negative mode, nitrogen temperature 300 ºC, flow 9 L/min, pressure of 310.2 KPa nebulization (45 psi) and potential difference 4000V. The elution mixture is shown in Table 3, where A = acetonitrile and B. 0.1% formic acid.

Table 3

| Time (min) | % de A |
|---|---|
| 0 | 10 |

(continued)

| Time (min) | % de A |
|---|---|
| 5 | 25 |
| 8 | 35 |
| 15 | 60 |
| 17 | 35 |
| 20 | 10 |

Then the chemical structure of the main molecules identified in the analysis is shown. The main compounds were detected: vanillic acid, chlorogenic acid, galoiquinico acid, gallic acid, luteolin and digaloiglucosa. In the state of the art, they have not reported chlorogenic acid, galoilquinico and digaloilglucosa acid in this species.

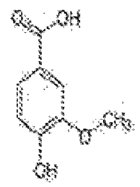

Vanilic acid

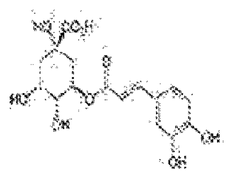

Clorogenic acid

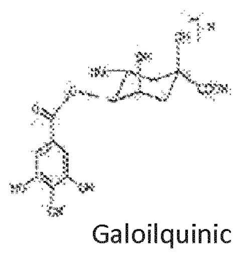

Galoilquinic acid

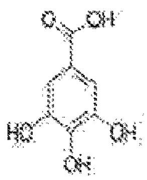

Gallic acid

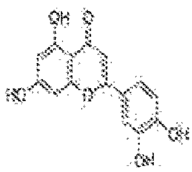

luteoline

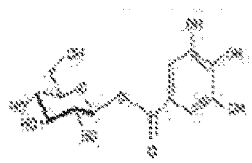

Digaloil -glucose

## Claims

**1.** A natural composition based on seedlings *Aristotelia chilensis* (Maqui) with antioxidants and antibacterial properties, **characterized in that**:

a) the plant material is obtained by *in vitro* culture of buds of adult trees,
b) a hydroalcoholic extract of plants grown *in vitro* is obtained for 20-45 days of culture and
c) an extract where a hydroalcoholic solution between 25% and 85% v/v of alcohohagua used.

**2.** Composition according to claim 1, wherein the extract is obtained by diffusion or maceration.

**3.** Composition according to claim 1, wherein the extraction is between 20 and 70 ºC.

**4.** Composition according to claims 1, 2, and 3, comprising vanillic acid, chlorogenic acid, galoiquinico acid, gallic acid, luteolin and digaloiglucosa.

**5.** Composition according to claim 4, wherein the extract has antioxidant properties.

**7.** Composition according to claim 4, **characterized in that** the extract can be encapsulated into cyclodextrins, maltodextrosas and acacia.

**8.** Composition according to any of the preceding claims, wherein the extract can be added to food, cosmetics and pharmaceuticals in a proportion of between 0.01 and 0.5% by weight.

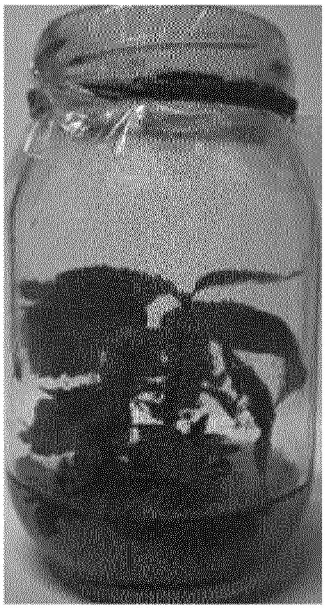

FIG 1

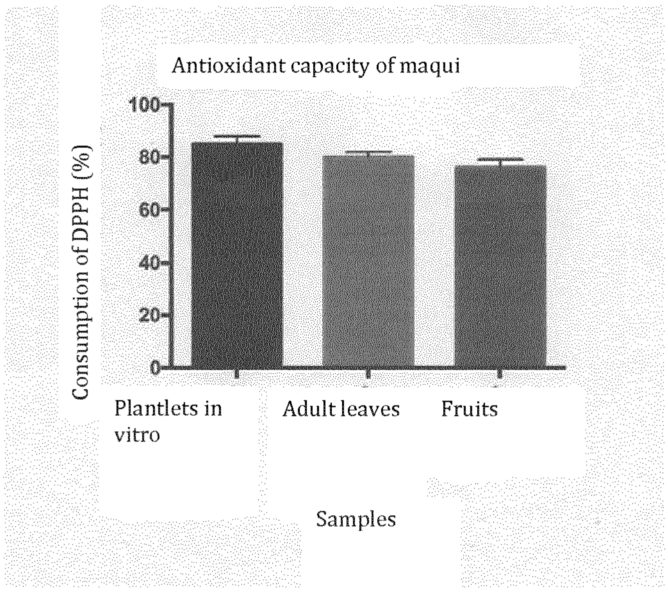
Antioxidant capacity of maqui
100
80
60
40
20
0
Consumption of DPPH (%)
Plantlets in vitro
Adult leaves
Fruits
Samples

FIG 2

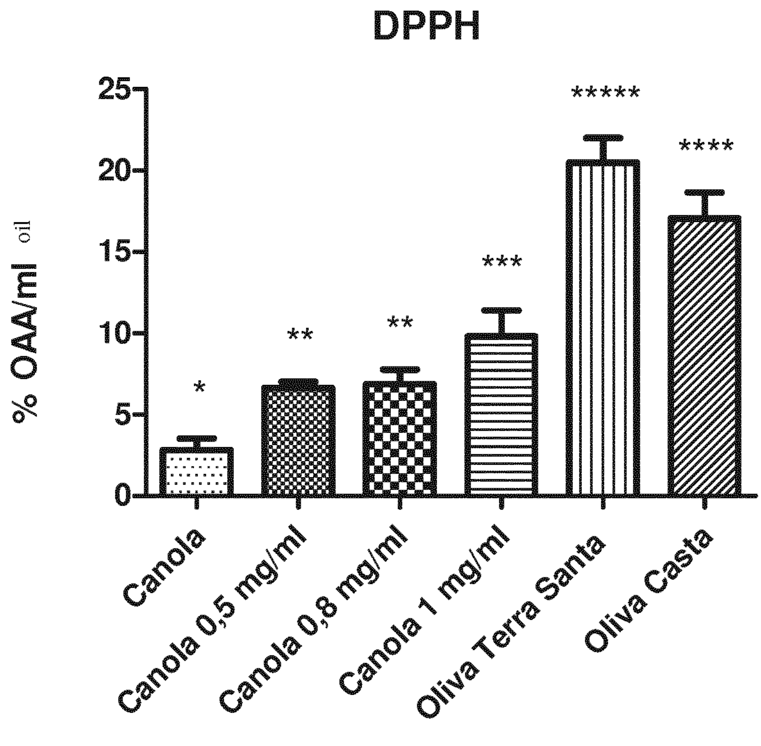
DPPH
% OAA/ml oil
25
20
15
10
5
0
*
**
**
***
*****
****
Canola
Canola 0,5 mg/ml
Canola 0,8 mg/ml
Canola 1 mg/ml
Oliva Terra Santa
Oliva Casta

FIG 3

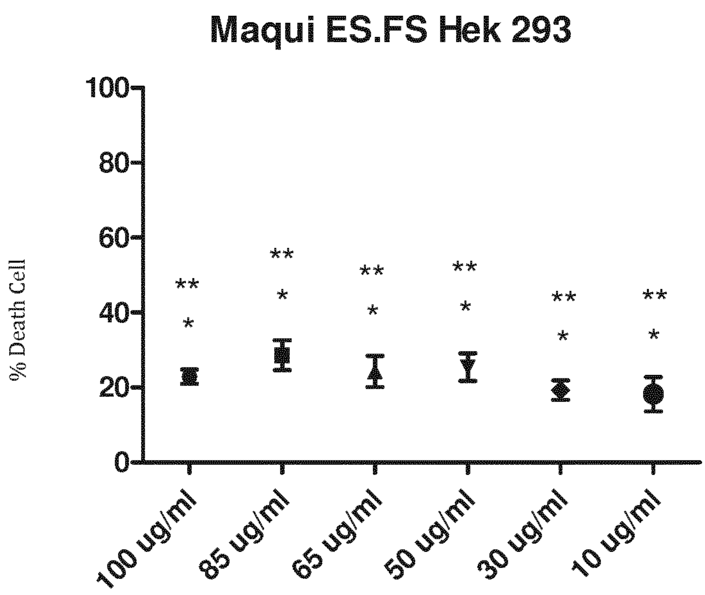
Maqui ES.FS Hek 293
% Death Cell
100
80
60
40
20
0
**
*
100 ug/ml
85 ug/ml
65 ug/ml
50 ug/ml
30 ug/ml
10 ug/ml

FIG 4

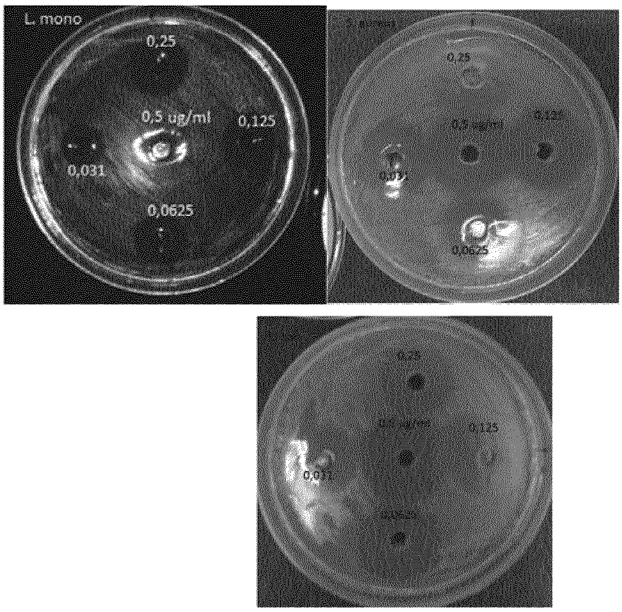
L. mono
0,25
0,5 ug/ml
0,125
0,031
0,0625
0,25
0,125
0,0625
0,25
0,125
0,031
0,0625

FIG 5

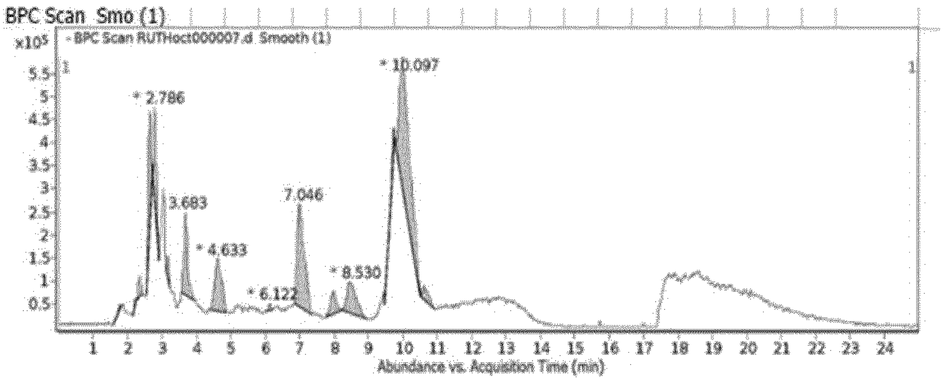
BPC Scan Smo (1)
- BPC Scan RUTHoct000007.d Smooth (1)
x10^5
* 2.786
3.683
* 4.633
* 6.122
7.046
* 8.530
* 10.097
Abundance vs. Acquisition Time (min)

FIG 6

**INTERNATIONAL SEARCH REPORT**

International application No.
PCT/CL2016/050081

A. CLASSIFICATION OF SUBJECT MATTER

(CIP): A61K36/185 (2017.01)

According to International Patent Classification (IPC) or to both national classification and IPC

B. FIELDS SEARCHED

Minimum documentation searched (classification system followed by classification symbols)

(CIP): A61K36/185

Documentation searched other than minimum documentation to the extent that such documents are included in the fields searched

Electronic data base consulted during the international search (name of data base and, where practicable, search terms used)

STN (BIOSIS, PCTFULL, USFULL), PubMed, Google Scholar, INAPI

C. DOCUMENTS CONSIDERED TO BE RELEVANT

| Category* | Citation of document, with indication, where appropriate, of the relevant passages | Relevant to claim No. |
|---|---|---|
| X | VIDAL, J. et al. Microencapsulation of maqui (Aristotelia chilensis [Molina] Stuntz) leaf extracts to preserve and control antioxidant properties. Chulean journal of agricultura! research, 2013, 73(1 ), 17-23. DOI : 10.4067/S0718-58392013000100003 The whole document | 1-5 and 7 |
| X | AVELLO, M. et al. Extractos antioxidantes y antimicrobianos de Aristotelia chilensis y Ugni molinae y sus aplicaciones como preservantes en productos cosméticos, [en línea] Boletín Latinoamericano y del Caribe de Plantas Medicinales y Aromáticas 2009, 8(6): 479-486. [Recuperado el 15-03-2017]. Recuperado de internet:<URL: ttp://www.redalyc.org/articulo.oa?¡d=85617461004> The whole document | 1-5 and 8 |
| A | CESPEDES, C. et al. Cultivo in vitro de Aristotelia chilensis (Mol.) Stuntz Elaeocarpaceae. Gayana Botánica, 1995, 52(2): 77-82. ISSN:0016-5301 The whole document | |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16/03/2017 16 March 2017 | 25/04/2017 25 April 2017 |
| Name and mailing address of the ISA/ INAPI Av. Libertador Bernardo O'Higgins 194, Piso 17 Santiago, Chile Facsimile No. | Authorized officer GARRIDO GAMBOA, Carolina Telephone No. 56-2-28870551 56-2-28870550 |

Form PCT/ISA/210 (second sheet) (January 2015)

**INTERNATIONAL SEARCH REPORT**

International application No.
PCT/CL2016/050081

C (Continuation). DOCUMENTS CONSIDERED TO BE RELEVANT

| Category* | Citation of document, with indication, where appropriate, of the relevant passages | Relevant to claim No. |
|---|---|---|
| A | M0LGAARD, P. et al. Antimicrobial evaluation of Huilliche plant medicine used to treat wounds. Journal of Ethnopharmacology, October 201 1 , 138(1 ): 219-227. doi: 10.1016/j.jep.201 1.09.006. Table 2, page 222. | |
| A | PEÑA, J. Aristotelia chilensis: A Possible Nutraceutical or Functional Food. Med chem, August 2015, 5:378-382. DOI: 10.4172/2161 -0444.1000289 The whole document | |
| A | RODRÍGUEZ, K. et al. Changes in bioactive components and antioxidant capacity of maqui, Aristotelia chilensis [Mol] Stuntz, berries during drying. LWT - Food Science and Technology, January 2016, 65: 537-542. Disponible online: 22 August 2015. DOI:10.1016/j.lwt.2015.08.050. The whole document | |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Antioxidants, from spices and herbs. **NAKATANI, N.** Natural Antioxidants: Chemistry, Health Effects and Applications. AOCS Press, 1997, 64-75
- **LOURDES B. ; GARCIA L ; RED D. ; ZANCHES E.** *Plants with antioxidant properties Biomedica Cuban Journal Research,* 2001, vol. 20, 231-235
- **M. AVELLO ; SUWALSKY M.** *Free radicals, natural antioxidants and protection mechanisms Athena,* 2006, vol. 492, 161-172
- **DZIEZAK, JD.** Antioxidants The ultimate answer Food Technol 40 to oxidation. *Preservatives,* 1986, 94-97
- **SHAHIDI, F. ; NACZK, M.** Phenolics in the food and nutraceuticals. CRC Press, 2004, 1-16
- **GUTIERREZ, J. ; RODRIGUEZ, G. ; BARRY-RYAN, C ; BOURKE, P.** *Efficacy of plant,* 2008
- **HULANKOVA, R. ; BORILOVA, G. ; STEINHAUSEROVA, I.** Combined antimicrobial effect of oregano essential oil and caprylic acid in minced beef. *meat Science,* 2013, vol. 95 (2), 190-194
- **FERNANDEZ-PAN, I. ; MENDOZA, M. ; MATE, JI.** Whey protein Isolate edible films With essential oils incorporated to improve increase the microbial quality of poultry. *J Sci Food Agric,* 2013, vol. 93 (12), 2986-2994
- **RODRIGUEZ, R.** Flora of Chile. 1995, 408
- **VALDEBENITO, G.** maqui Package Technology. Forestry Institute, 10 June 2012
- Study of chemical species Eleocarpaceae family grown in Chile. **SILVA, M. ; BITTNER, M.** Chemistry flora Orlando Muñoz Chile. 1992